# EUROPEAN PATENT SPECIFICATION

(11) **EP 1 277 440 B1**
(45) Date of publication and mention of the grant of the patent: **15.10.2003**
(21) Application number: 01830452.7
(22) Date of filing: 04.07.2001
(51) Int. Cl.: A61B 10/00

(54) **Bone biopsy device and process for making the same**
Knochenbiopsievorrichtung und zugehöriges Herstellungsverfahren
Dispositif pour biopsie medullaire et procédé de fabrication correspodant

(43) Date of publication of application: 22.01.2003
(73) Proprietor: Zambelli, Flavio Augusto, 20017 RHO (Milano) (IT)
(72) Inventor: Zambelli, Flavio Augusto, 20017 RHO (Milano) (IT)
(74) Representative: Ghioni, Carlo Raoul Maria

(56) References cited:
- DE-U- 20 010 879
- US-A- 4 838 282
- US-A- 5 331 972
- US-A- 5 634 473
- US-A- 6 022 324
- US-A- 6 110 128

## Description

The present invention relates to a needle for carrying out bone biopsies and to a process for making the cannula of said needle.

It is known that bone biopsy consists in taking a sample of a fragment of bone marrow from a living being and examining it, for diagnostic purposes.

In particular, on human beings taking samples is usually carried out under local anaesthesia by use of an appropriate needle.

For the purpose, needles for bone biopsy are known which are formed of a steel tube, or cannula, within which an extractable stem, made of steel too and called stylet, is placed.

The stylet has a tip that, when the stylet itself is inserted in the cannula, projects from the cannula and serves to perforate the outer bone layer which is harder and more compact.

The cannula and stylet are respectively provided with a handgrip. When the two elements are associated, the handgrips form a single body to enable a firm hold by an operator.

The needle is inserted in the bone by perforating the compact bone portion with the stylet tip, then the stylet is removed from the cannula and the cannula moves farther into the bone.

The further moving forward of the cannula enables dissection of a barrel-shaped fragment of bone marrow which occupies a place in the first portion of the cannula itself.

In order to separate the barrel-shaped fragment from the remaining marrow portion, the operator carries out a series of oscillatory movements entailing inclination of the cannula relative to the penetration direction, so as to separate the barrel-shaped fragment at the base.

This type of needle does not always ensure a successful operation. Often the operation fails at the moment of drawing the cannula out of the bone. In fact, when the cannula is drawn out, the sample may slip off the latter and stay in its original seat.

Also known are needles for bone biopsy provided with cannulas in which the end of the portion to be inserted into the bone is of a conical shape.

Tapering of the inner wall of the cannula enables the barrel-shaped fragment being dissected, following an oscillatory motion, to take a sloping position and lay down on the wall itself.

The position taken by the sample, as a result of the particular conformation of the cannula, would prevent the barrel-shaped fragment from slipping off the instrument with ease.

This however does not happen under all circumstances because sometimes during the extraction step, the barrel-shaped fragment slips along the inner sloping surface of the cannula tip and stays within the bone.

A further improvement was achieved by adopting a needle provided with a cannula with a cone-shaped end also having an annular groove formed in the inner wall of the tapering portion.

The barrel-shaped fragment separated from the marrow deposits in the cone taking an interference position with the groove. Jamming of the barrel-shaped fragment in the groove offers an additional certainty of success during the extraction step.

At all events, since the groove is formed by material removal working, it has a sloping surface with an inclination angle corresponding to the conicity of the cutter tip used for carrying out the operation.

In this case too, however, the barrel-shaped fragment has a tendency to slip along said sloping surface, although to a smaller extent.

Therefore, while ensuring a greater percentage of successful operations, the needles of this type do not give a full certainty of success.

It is also known from document US 5 634 473 a biopsy needle having an outer cannula, an inner tube and a stylet removably inserted inside the inner tube and inside the cannula. The distal end of the inner tube is provided with a snare in the form of a coil which, when the inner tube is positioned in the cannula, is located against a shoulder provided on the interior of the outer cannula at a position where the diameter changes. Upon rotation of the inner tube with respect to the outer cannula, the coil decreases in diameter to hold the biopsy piece within the outer needle. This document forms the preamble of independent claim 1.

The Applicant has found that needles of the above described type can be further improved from different points of view, in particular as regards success in carrying out the operation, quality of the bioptic sample taken, and reduction of traumas on patients.

In fact, in addition to the drawback of a high number of unsuccessful operations, the above mentioned prior art needles involve a big trauma for patients, first due to the necessity of carrying out an oscillatory motion to separate the sample and, in addition, due to the fact that the cone-ended cannula has a maximum diameter much bigger than the stylet diameter.

The hole created by the stylet tip is therefore enlarged during penetration of the cannula, which will require a great effort by the operator and will cause a more traumatic injury on the patient.

Finally, still due to the oscillatory motion, the bioptic sample may appear damaged and not very compact.

Therefore, the present invention aims at accomplishing a needle for bone biopsy capable of solving the above mentioned drawbacks.

In particular, it is an object of the present invention to make a needle capable of ensuring that all operations carried out will be successful.

It is another object of the present invention to make a needle enabling traumas on patients to be reduced.

It is a further object of the present invention to make a needle for bone biopsy by which sampling will be simpler and quicker.

A still further object of the present invention is to make a needle enabling large and compact samples to be taken.

Finally the present invention aims at providing a simple and cheap process for making a needle cannula for bone biopsy of the above mentioned type.

The foregoing and further objects that will become more apparent in the course of the following description are achieved by a needle for bone biopsy comprising the features set forth in the characterizing portion of claim 1 and in the claims depending thereon.

It is also an object of the present invention to provide a process for making a cannula of a needle for bone biopsy comprising the features set forth in claim 6 and in the claims depending thereon.

Further features and advantages will be best understood from the detailed description of a preferred but not exclusive embodiment of a needle for bone biopsy in accordance with the present invention. This description will be taken hereinafter with reference to the accompanying drawings, in which:

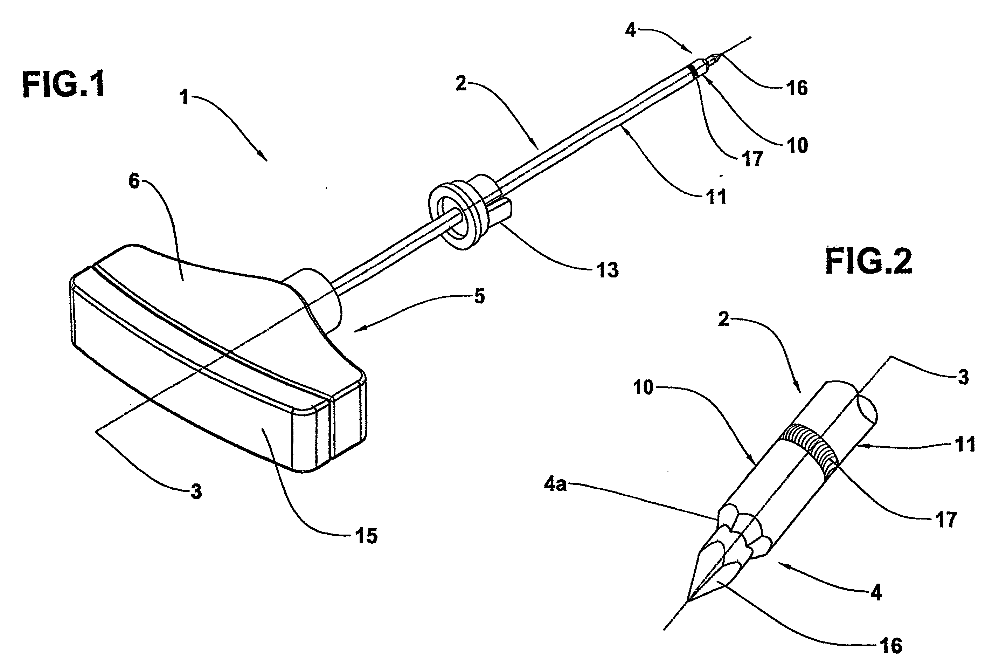

- Fig. 1 is a perspective view of a needle for bone biopsy, made in accordance with the present invention;
- Fig. 2 is an enlarged view of the end of the needle shown in Fig. 1;

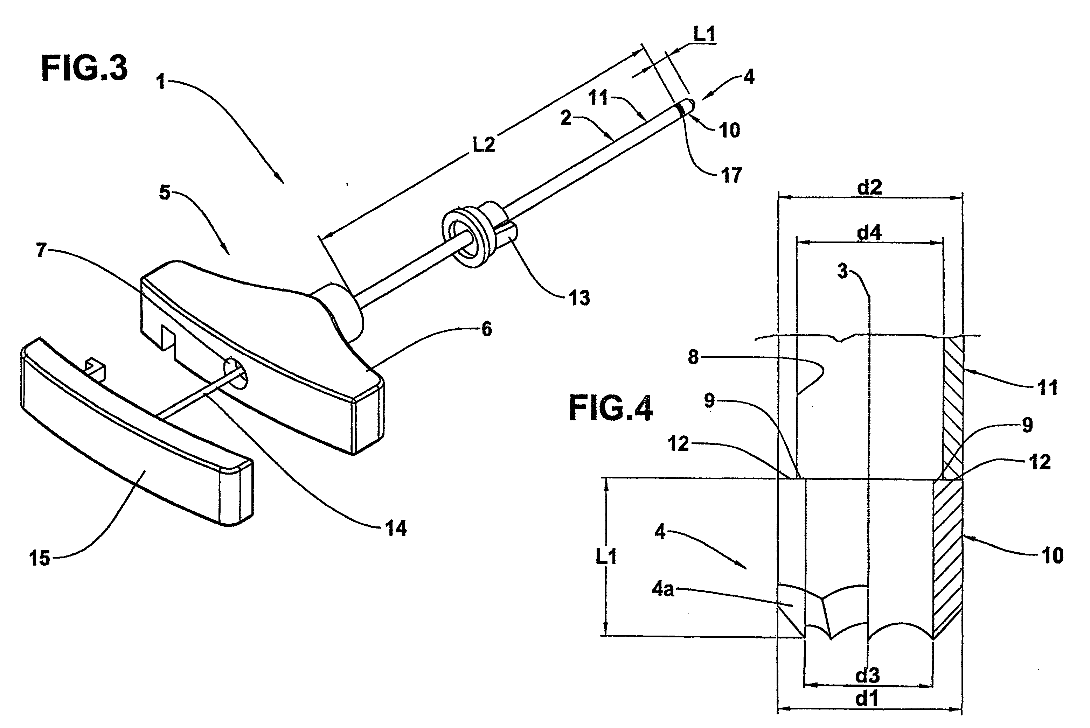

- Fig. 3 is a perspective view of the needle shown in Fig. 1 with a stylet partly unthreaded from the cannula;
- Fig. 4 is an enlarged view partly in section of the end of the needle cannula shown in Fig. 1.

With reference to the drawings, a needle for bone biopsy in accordance with the present invention has been generally identified by reference numeral 1.

Needle 1 comprises a cannula 2 of a rectilinear axis 3 and usually of a length included between 15 and 20 centimetres and an outer diameter d1, d2 in the order of few millimetres.

Cannula 2 has a first free end 4 and a second end 5 provided with a first handgrip 6 surrounding the cannula 2 itself and leaving the entrance 7 of the second end 5 free.

Preferably, cannula 2 has a constant outer diameter d1.

Advantageously, the inner side surface 8 of cannula 2 has an annular portion defining a surface 9 perpendicular to the axis 3 of cannula 2 and placed close to the first end 4, a few millimetres therefrom.

Portion 9 faces towards the second end 5 of cannula 2 and preferably extends ring-like on the inner surface 8 so as to cause narrowing of the passage area from the second 5 to the first end 4.

Such a portion 9 constitutes an abutment or stop element for the portion of the bioptic barrel-shaped fragment associated therewith.

In accordance with a preferred embodiment, cannula 2 is made up of a first 10 and a second 11 cylindrical bodies disposed in coaxial alignment.

Advantageously, the two cylindrical bodies 10, 11 have the same outer diameter d1, d2.

The first cylindrical body 10 has a length L1 in the order of few millimetres whereas the second body 11 forms the rest of cannula 2 and its length L2 is substantially coincident with the length of the cannula itself.

In addition, the first cylindrical body 10 has an inner diameter d3 lower than the inner diameter d4 of the second cylindrical body 11 and preferably the inner diameter d3 of the first cylindrical body 10 is constant over the body length L1.

In this way, portion 9 which is perpendicular to axis 3 is defined between the first 10 and second 11 cylindrical bodies.

More specifically, portion 9 is made up of the fraction of the annular base 12 of the first cylindrical body 10 facing towards the inside of the second cylindrical body 11.

Advantageously, the first end 4 has a sharp edge 4a to enable penetration of cannula 2 into the medullary tissue.

Placed around cannula 2 and sliding thereon is a cylinder 13 which can be locked on cannula 2 to indicate which depth can be reached.

A stem or stylet 14 is inserted in cannula 2, in coaxial relationship therewith, and it can be freely removed by slipping it off from entrance 7.

A second handgrip 15 is fastened to stylet 14 and is associated with the first handgrip 6 when the stylet is completely inserted in cannula 2.

Stylet 14 is further provided, on its end opposite to the second handgrip 10, with a perforating tip 16. When stylet 14 is inserted in cannula 2, the tip 16 projects from the first end 4 of cannula 2 and constitutes the needle point 1.

Needle 1 with this configuration is introduced into the bone until it perforates the compact part of same; subsequently stylet 14 is drawn out of cannula 2 and, by grasping the first handgrip 6, the operator causes cannula 2 alone to further move forward in the soft bone part so that the sharp edge 4a dissects a cylindrical marrow fragment.

Thus the marrow fragment is disposed within cannula 2, partly close to the first end 4 and partly farther where the inner diameter d4 is greater.

Since the marrow is spongy and elastic, the portion of the cylindrical fragment occupying the region of greater diameter d4 tends to widen out, partly overlapping portion 9 perpendicular to axis 3 of cannula 2 which therefore surely prevents escape of the fragment while cannula 2 is pulled out of the bone.

In addition, resting of the fragment on portion 9 perpendicular to axis 3 is of such a nature that separation of said fragment from the rest of the marrow takes place during extraction, without prior oscillation of cannula 2 still inserted in the bone being required.

After describing the invention from a structural and operating point of view, a process for making a needle cannula for bone biopsy in accordance with the present invention is now disclosed.

Cannula 2 is made by disposing a first cylindrical body 10 and a second elongated cylindrical body 11 close to each other in coaxial relationship, said bodies preferably having the same outer diameter d1, d2 and different inner diameters d3, d4.

In particular the inner diameter d3 of the first cylindrical body 10 is smaller than that d4 of the second cylindrical body 11.

In addition, the length L1 of the first cylindrical body 10 is comparable to the respective outer diameter d1, whereas the length L3 of the second body 11 is remarkably greater.

The two cylindrical bodies 10. 11 disposed in side by side relationship are mutually fastened by welding so that a weld bead 17 ensures a tight seal.

Preferably, in order to limit overheating of the two bodies that could lead to undesirable deformations, a laser welding is carried out, by submitting the ends of the two bodies disposed close to each other to a high-intensity light beam.

Finally, sharpening of the edge 4a of cannula 2 placed on the first cylindrical body 10 is carried out.

The present invention achieves important advantages.

First of all the needle in accordance with the present invention ensures removal of a marrow sample during every operation carried out. As a proof of such assertion table 1 is annexed hereto.

Reproduced therein are the results concerning a comparative bioptic test consisting of twenty sample-drawing operations or sample-drawing attempts carried out on an ox bone using a traditional needle and a needle in accordance with the present invention.

In particular, reproduced in the table columns are the lengths of the bioptic sample extracted at each sampling carried out at a depth of about 20 millimetres; at the bottom of each column there is indicated the sum total of the lengths of the extracted samples.

Column 1 reproduces the results obtained with a needle provided with a traditional cannula, i.e. with a cone-shaped end and an annular groove, and without carrying out oscillation of the needle before extraction.

Column 2 reproduces the results obtained with a needle provided with a traditional cannula and with execution of needle oscillation before extraction.

Column 3 reproduces the results obtained with a needle provided with a cannula in accordance with the present invention and without carrying out any oscillation of the needle before extraction.

It should be pointed out first of all that the total length of the samples extracted with a needle in accordance with the present invention is about twice that of the samples extracted with a traditional needle.

In addition, it will be recognized that all operations carried out with a needle in accordance with the present invention led to a positive result whereas with the traditional needle a certain percentage of unsuccessful attempts occurred.

Finally, the average length of the samples extracted with a needle according to the present invention is longer than the average length of the samples extracted with a traditional instrument.

Therefore, the invention also enables large and compact samples to be obtained.

Furthermore, it also enables traumas generated on patients to be reduced, both because the cannula has a diameter only slightly bigger than the diameter of the stylet tip, and because the sampling operation takes place with a net pull without oscillations being required to separate the osseous fragment.

The needle according to the present invention also ensures a greater ease and quickness in carrying out the operation.

Finally, the process for making a needle cannula for bone biopsy in accordance with the present invention allows the cannula production time and costs to be reduced.

The device as conceived is susceptible of many modifications and variations, all falling within the scope of the appended claims. All of the details can be replaced by other technically equivalent elements and practically all materials used and sizes can be of any nature and magnitude depending on requirements.

**Table 1**

| Test No. | Traditional needle. Extraction without oscillation. Sample length (mm). | Traditional needle. Extraction with oscillation. Sample length (mm). | Invention. Extraction without oscillation. Sample length (mm) |
|---|---|---|---|
| 1 | 8 | 15 | 19 |
| 2 | 0 | 0 | 20 |
| 3 | 0 | 0 | 18 |
| 4 | 15 | 2 | 15 |
| 5 | 16 | 15 | 19 |
| 6 | 10 | 9 | 10 |
| 7 | 13 | 10 | 17 |
| 8 | 3 | 11 | 13 |
| 9 | 3 | 19 | 20 |
| 10 | 0 | 7 | 27 |
| 11 | 21 | 26 | 23 |
| 12 | 19 | 19 | 24 |
| 13 | 0 | 0 | 19 |
| 14 | 5 | 19 | 22 |
| 15 | 0 | 12 | 18 |
| 16 | 0 | 0 | 17 |
| 17 | 0 | 0 | 19 |
| 18 | 12 | 11 | 11 |
| 19 | 14 | 2 | 17 |
| 20 | 0 | 12 | 25 |
| Total (mm) | **139** | **189** | **373** |

## Claims

1. A needle for body biopsy, comprising:
- a cannula (2) having a first end (4), a second end (5) and an inner side surface (8);
- a stem or stylet (14) removably inserted in the cannula (2) and coaxial with said cannula (2); said stylet (14) having a perforating tip (16) projecting from the first end (4) of the cannula (2);
- a first handgrip (6) placed around the cannula (2) and at the second end (4) of said cannula (2);
- a second handgrip (15) fastened to the stylet (14) and associated with the first handgrip (6);
**characterized in that** the cannula comprises a first cylindrical body (10) and a second cylindrical body (11) disposed close to the first body (10) in coaxial relationship therewith; the first cylindrical body (10) having an inner diameter (d3) smaller than the inner diameter (d4) of the second cylindrical body (11) and defining, on the inner side surface (8) close to the first end (4) of said cannula (2)and between the first (10) and the second (11) cylindrical bodies, an annular portion showing a surface (9) perpendicular to the longitudinal axis (3) of the cannula (2) and facing towards the second end (5).

2. A needle as claimed in claim 1, **characterized in that** the cannula (2) further comprises a weld bead (17) disposed between the first (10) and second (11) cylindrical bodies.

3. A needle as claimed in claim 1, **characterized in that** the first cylindrical body (10) has a constant inner diameter (d3).

4. A needle as claimed in claim 1, **characterized in that** the first cylindrical body (10) has an outer diameter (d1) which is the same as the outer diameter (d2) of the second cylindrical body (11).

5. A needle as claimed in claim 1, **characterized in that** the first end (4) of the cannula (2) has a sharp edge (4a).

6. A process for making a needle cannula for bone biopsy, **characterized in that** it comprises the following steps:
- making a first cylindrical body (10);
- making a second elongated cylindrical body (11) having an inner diameter (d4) bigger than the inner diameter (d3) of the first cylindrical body (10);
- moving the first body (10) into coaxial side by side relationship with the second body (11);
- fastening the first body (10) to the second body (11) to form a cannula (2) having an inner side surface (8); said inner side surface (8) having an annular portion disposed between the first body (10) and second body (11) and defining a surface (9) perpendicular to the longitudinal axis (3) of the cannula (2).

7. A process as claimed in claim 6, **characterized in that** the step of fastening the first body (10) to the second body (11) consists in welding said first body (10) to said second body (11).

8. A process as claimed in claim 7, **characterized in that** the step of welding said first body (10) to said second body (11) consists in submitting the ends of the two bodies (10, 11) disposed side by side to a laser beam.

9. A process as claimed in claim 6, **characterized in that** it further comprises the step of sharpening the edge (4a) of the cannula (2) disposed on the first cylindrical body (10).

## Patentansprüche

1. Knochenbiopsienadel, umfassend:
- eine Kanüle (2) mit einem ersten Ende (4), einem zweiten Ende (5) und einer inneren Seitenfläche (8);
- einen Schaft oder Stift (14), der in der Kanüle (2) entfernbar eingesetzt und zur Kanüle (2) koaxial ist, wobei der Schaft (14) eine Bohrspitze (16) aufweist, die vom ersten Ende (4) der Kanüle vorsteht (2);
- einen ersten Griff (6), der um die Kanüle (2) herum und im Bereich des zweiten Endes (5) der Kanüle (2) angeordnet ist;
- einen zweiten Griff (15), der am Schaft (14) befestigt und dem ersten Griff (6) zugeordnet ist;
**dadurch gekennzeichnet, dass** die Kanüle einen ersten, zylindrischen Körper (10) und einen dem ersten Körper (10) koaxial anliegenden zweiten zylindrischen Körper (11) umfasst; wobei der erste zylindrische Körper (10) einen Innendurchmesser (d3) besitzt, der kleiner ist als der Innendurchmesser (d4) des zweiten zylindrischen Körpers (11) und auf der inneren Seitenfläche (8) in der Nähe des ersten Endes (4) der Kanüle (2) und dem ersten (10) und dem zweiten (11) zylindrischen Körper einen ringförmigen Abschnitt festlegt, der eine zur Längsachse (3) der Kanüle (3) senkrechte Oberfläche (8) aufweist und dem zweiten Ende (5) gegenüberliegt.

2. Nadel nach Anspruch 1, **dadurch gekennzeichnet, dass** die Kanüle (2) überdies eine Schweißnaht (17) umfasst, die zwischen dem ersten (10) und dem zweiten (11) zylindrischen Körper angeordnet ist.

3. Nadel nach Anspruch 1, **dadurch gekennzeichnet, dass** der erste zylindrische Körper (10) einen konstanten Innendurchmesser (d3) aufweist.

4. Nadel nach Anspruch 1, **dadurch gekennzeichnet, dass** der erste zylindrische Körper (10) einen Außendurchmesser (d1) aufweist, der gleich dem Außendurchmesser (d2) des zweiten zylindrischen Körpers (11) ist.

5. Nadel nach Anspruch 1, **dadurch gekennzeichnet, dass** das erste Ende (4) der Kanüle (2) eine scharfe Kante (4a) aufweist.

6. Verfahren zur Herstellung einer Kanüle einer Knochenbiopsienadel, **dadurch gekennzeichnet, dass** es folgende Schritte umfasst:
- Ausführen eines ersten zylindrischen Körpers (10);
- Ausführen eines zweiten, länglichen, zylindrischen Körpers (11), der einen Innendurchmesser (d2) aufweist, der größer als der Innendurchmesser (d3) des ersten zylindrischen Körpers (10) ist;
- koaxiales Annähern des ersten Körpers (10) an den zweiten Körper (11);
- Befestigen des ersten Körpers (10) am zweiten Körper (11) zur Bildung einer Kanüle (2) mit einer inneren Seitenfläche (8);
wobei die innere Seitenfläche (8) einen winkelförmigen Abschnitt aufweist, der zwischen dem ersten Körper (10) und dem zweiten Körper (11) liegt und eine Oberfläche (9) festlegt, die zur Längsachse (3) der Kanüle (2) senkrecht ist.

7. Verfahren nach Anspruch 6, **dadurch gekennzeichnet, dass** der Schritt des Befestigens des ersten Körpers (10) am zweiten Körper (11) im Schweißen des ersten Körpers (10) am zweiten Körper (11) besteht.

8. Verfahren nach Anspruch 7, **dadurch gekennzeichnet, dass** der Schritt des Schweißens des ersten Körpers (10) am zweiten Körper (11) im Vorelgen der angenäherten Enden der beiden Körper (10, 11) einem Laserstrahl besteht.

9. Verfahren nach Anspruch 6, **dadurch gekennzeichnet, dass** es überdies den Schritt umfasst, die am ersten zylindrischen Körper (10) angesetzte Kante (4a) der Kanüle (2) zu schärfen.

## Revendications

1. Aiguille pour biopsie médullaire, comprenant:
- une canule (2) ayant une première extrémité (4), une deuxième extrémité (5) et une surface latérale intérieure (8);
- une tige ou stylet (14) inséré de manière amovible dans la canule (2) et coaxial avec ladite canule (2); ledit stylet (14) ayant une pointe perforatrice (16) faisant saille de la première extrémité (4) de la canule (2);
- une première poignée (6) mise autour de la canule (2) et en correspondance avec la deuxième extrémité (4) de ladite canule (2);
- une deuxième poignée (15) fixée au stylet (14) et associée à la première poignée (6);
**caractérisée en ce que** la canule comporte un premier corps cylindrique (10) et un deuxième corps cylindrique (11) disposé à proximité du premier corps (10) et coaxial avec ce dernier; le premier corps cylindrique (10) ayant un diamètre intérieur (d3) plus petit que le diamètre intérieur (d4) du deuxième corps cylindrique (11) et définissant, sur la surface latérale intérieure (8) à proximité de la première extrémité (4) de ladite canule (2) et entre le premier (10) et le deuxième (11) corps cylindriques, une portion annulaire présentant une surface (9) perpendiculaire à l'axe longitudinal (3) de la canule (2) et orientée vers la deuxième extrémité (5).

2. Aiguille selon la revendication 1, **caractérisée en ce que** la canule (2) comporte en outre un cordon de soudure (17) disposé entre les premier (10) et deuxième (11) corps cylindriques.

3. Aiguille selon la revendication 1, **caractérisée en ce que** le premier corps cylindrique (10) a un diamètre intérieur constant (d3).

4. Aiguille selon la revendication 1, **caractérisée en ce que** le premier corps cylindrique (10) présente un diamètre extérieur (d1) égal au diamètre extérieur (d2) du deuxième corps cylindrique (11).

5. Aiguille selon la revendication 1, **caractérisée en ce que** la première extrémité (4) de la canule (2) a un bord tranchant.

6. Procédé pour la fabrication de la canule d'une aiguille pour biopsie médullaire, **caractérisé en ce qu'**il comporte les étapes suivantes:
- fabrication d'un premier corps cylindrique (10);
- fabrication d'un deuxième corps cylindrique allongé (11) présentant un diamètre intérieur (d4) plus long que le diamètre intérieur (d3) du premier corps cylindrique (10);
- déplacement du premier corps (10) en position rapprochée d'une façon coaxiale du deuxième corps (11);
- fixation du premier corps (10) au deuxième corps (11) à former une canule (2) ayant une surface latérale intérieure (8); ladite surface latérale intérieure (8) présentant une portion annulaire disposée entre le premier corps (10) et le deuxième corps (11) et définissant une surface (9) perpendiculaire à l'axe longitudinal (3) de la canule (2).

7. Procédé selon la revendication 6, **caractérisé en ce que** l'étape de fixer le premier corps (10) au deuxième corps (11) comporte le soudage dudit premier corps (10) audit deuxième corps (11).

8. Procédé selon la revendication 7, **caractérisé en ce que** l'étape de souder ledit premier corps (10) audit deuxième corps (11) prévoit de soumettre les extrémités rapprochées des deux corps (10, 11) à un rayon laser.

9. Procédé selon la revendication 6, **caractérisé en ce qu'**il comporte en outre l'étape de rendre tranchant le bord (4a) de la canule (2) disposé sur le premier corps cylindrique (10).
